# EUROPEAN PATENT APPLICATION

(11) **EP 4 520 249 A1**
(43) Date of publication of application: **12.03.2025**
(21) Application number: 24197365.0
(22) Date of filing: 29.08.2024
(51) Int. Cl.: A61B 1/00, A61B 1/015

(54) **ENDOSCOPE COMPRISING A SUCTION VALVE**

(30) Priority: 05.09.2023 EP 23195491
(71) Applicant: Ambu A/S, 2750 Ballerup (DK)
(72) Inventor: MORTON, Alistair David, 2300 Copenhagen (DK)
(74) Representative: Winter, Brandl - Partnerschaft mbB

(57) **Abstract**

The present disclosure relates to an endoscope (2) comprising a proximal endoscope handle or interface (4). The endoscope handle or interface (4) comprises a suction valve (26) and a housing, which comprises a first half-shell (36a) and a second half-shell (36b). The suction valve (26) is mounted between the first half-shell (36a) and the second half-shell (36b). The first half-shell (36a) comprises a first, outer fixing portion (64) and a second, inner fixing (66) portion, wherein the first, outer fixing portion (64) is configured to receive a first portion of the suction valve (26) and wherein the second, inner fixing portion (66) is configured to receive a second portion of the suction valve (26). The second half-shell (36b) comprises a third, outer fixing portion (74) and a fourth, inner fixing portion (76), wherein the third, outer fixing portion (74) is configured to receive the first portion of the suction valve (26), and wherein the fourth, inner fixing portion (76) is configured to receive the second portion of the suction valve (26). The second, inner fixing portion (66) and the fourth inner fixing portion (76) have a different geometry, and the second, inner fixing portion (66) is configured to suppress at least one degree of freedom of the suction valve (26) and the fourth inner fixing portion (76) is configured to suppress a remaining degree or remaining degrees of freedom of the suction valve (26), for fixing the suction valve (26) in its position and orientation in the housing.

## Description

The present disclosure relates to an endoscope comprising a proximal endoscope handle or interface and an insertion cord extending from the endoscope handle or interface and configured to be inserted into a patient's body cavity, the endoscope having a suction valve mounted in the endoscope handle or interface. The present disclosure further relates to a method of assembling the endoscope and a system comprising an endoscope and a video processing apparatus (VPA).

### Related art

Endoscopes which include specialized instruments such as bronchoscopes, arthroscopes, colonoscopes, laparoscopes, gastroscopes, and duodenoscopes are well-known from the related art and are typically used for visual examination and diagnosis of hollow organs and body cavities, as well as to assist in surgery, e.g. for a targeted tissue sampling. Both reusable and disposable endoscopes are known from the related art. Known endoscopes usually comprise: an endoscope handle or interface; an insertion cord extending from the endoscope handle or interface, configured to be inserted into a patient's body cavity and comprising an insertion tube, a bending section and a distal tip unit; and a working channel. The working channel extends from the working channel access port at the endoscope handle to the distal tip unit.

When examining an object such as a body cavity or hollow organ with an endoscope it is desirable to have a clear and good view or visibility of the examined object. However, the visibility of such an object is often affected by undesirable fluid or mucus, which may obstruct visibility of the object to be examined. Furthermore, it may be desirable to remove tissue parts or particles during the surgery for example to extract a sample for further analysis, or to provide better visibility. It is thus desirable to remove such undesirable fluid or mucus, as well as such tissue parts or particles, using a suction device, such as a vacuum pump.

It is desirable that the surgeon can start and interrupt the suction at any time. Basically, it would be possible to control a vacuum pump performance for this purpose. However, this is associated with considerable delays caused by a startup and a shutdown of the pump motor.

In order to overcome these disadvantages, suction valves have become established in endoscopes, with which the effect of the suction power constantly applied by the vacuum pump can be applied or switched off. Further general requirements for such a suction valve may be that the suction valve is easy and quick to operate, and closes reliably and safely.

Various suction valves for endoscopes are known in the related art.

Basically, suction valves comprising a housing and a piston that is movable within the housing are well-known.

For example, EP 3 854 291 A1 discloses a suction valve assembly including a housing and a plunger, which moves upwards and downwards to open or close the suction valve.

US 2023/200632 A1 discloses a suction valve body with a suction valve button, which is used to control suction.

US 5 871 441 A discloses a suction valve that can be connected to a working channel of an endoscope and includes a preferably cylindrical housing, a piston, a spring and a button. The piston is provided with a flow channel and is movably accommodated within the housing. The button preferably on top of the piston is connected to the piston such that the piston is movable together with the button. An operator can press or push the button to move the piston from a valve closed state to a valve open state. The spring ensures that the valve is usually in the valve closed state, in particular when the operator does not press down the button. The housing is provided with an inlet opening that is connected to the working channel of the endoscope and with an outlet opening that is connected to a suction device. Usually, the suction device is active or running. When the valve is in the valve closed state an outer circumferential surface of the cylindrically shaped piston blocks the inlet opening. When the operator presses down the button, the piston is moved downwards such that the flow channel of the piston connects the inlet opening of the housing with the outlet opening of the housing, and a fluid flow through the suction valve is enabled. Hence, fluid or mucus or tissue parts or particles can be sucked from the working channel of the endoscope through the suction valve.

Suction valves known from the related art are usually securely and sealingly positioned and fixed in the housing of the endoscope handle to ensure a proper functioning of the endoscope.

In related art endoscopes and especially single-use endoscopes, the suction valve is usually glued into the housing of the handle. Such gluing involves considerable assembly work, which makes the endoscope more complex to manufacture and thus more expensive.

US 5 299 561 A discloses a configuration in which the suction valve is formed integrally with the housing and a valve spring is to be inserted or mounted into the monolithic housing. Such a monolithic design has the disadvantage that the complex geometry of the valve has to be formed directly in the housing, which significantly increases the complexity of the housing and thus the manufacturing costs.

### Brief description of the disclosure

The object of the present disclosure is therefore to overcome or at least reduce the disadvantages of the related art. More specifically, it is the purpose of the present disclosure to provide an endoscope having a suction valve, wherein the endoscope is formed from components that are simple and inexpensive to manufacture, and wherein the endoscope is easy to assemble.

This object is solved by an endoscope according to independent claim 1, a method according to claim 10 and system according to claim 11. Advantageous aspects and embodiments of the present disclosure are claimed in the dependent claims and/or are described below.

In detail, the present disclosure relates to an endoscope that comprises a proximal endoscope handle or interface, an insertion cord extending from the endoscope handle or interface and configured to be inserted into a patient's body cavity and a working channel configured to let fluid and mucus sucked from the patient's body cavity flow therethrough. The endoscope handle or interface comprises a housing and a suction valve configured to control a suction through the working channel and having a valve closed state and a valve open state. The housing comprises a first half-shell and a second half-shell. The suction valve comprises a valve body having an inlet connected to the working channel, an outlet provided and configured to be connected to a suction device and a stem movable axially in the valve body between at least a first position, in which the suction valve is in the valve closed state, and a second position, in which the suction valve is in the valve open state. The suction valve is mounted or mountable between the first half-shell and the second half-shell. The first half-shell comprises a first, outer fixing portion and a second, inner fixing portion. The first, outer fixing portion is configured to receive a first portion of the suction valve and the second, inner fixing portion is configured to receive a second portion of the suction valve.

Preferably, the second half-shell comprises a third, outer fixing portion and a fourth, inner fixing portion, wherein the third, outer fixing portion is configured to receive the first portion of the suction valve, and wherein the fourth, inner fixing portion is configured to receive the second portion of the suction valve.

Preferably, the second, inner fixing portion of the first half shell and the fourth inner fixing portion of the second half shell have a different geometry or a different shape.

Especially preferred, the second, inner fixing portion is configured to suppress or constrain at least one degree of freedom of the suction valve, in particular of the valve body of the suction valve, and the fourth, inner fixing portion is configured to suppress or constrain a remaining degree or remaining degrees of freedom of the suction valve, in particular of the valve body of the suction valve, for fixing the suction valve in its position and orientation in the housing of the endoscope handle or interface.

It is to be understood, that a body like the suction valve has six degrees of freedom in space, three rotational degrees of freedom and three translational degrees of freedom.

"Suppressing" a degree of freedom is to be understood as preventing an arbitrary translational or rotational movement of the suction valve in or relative to the housing of the endoscope at least in one direction along an axis of a Cartesian coordinate system or at least around an axis of the Cartesian coordinate system. "Suppressing" can be understood as "constraining".

It is to be understood, that the degrees of freedom of the suction valve refer to the suction valve as a whole and in particular to the valve body, however not to the stem. In other words, if degrees of freedom or movements of the suction valve are suppressed, this means a fixation of the suction valve and in particular of the valve body relative to the housing of the endoscope but does not mean that e.g. the stem may be prevented from moving.

The present disclosure relates to an endoscope, preferably a single use endoscope, comprising the endoscope handle or interface. In other words, the handle may be substituted by the interface. Alternatively, it is conceivable that the interface is formed on the handle. The handle may contain essential control elements, such as a lever for controlling bending of the endoscope for the endoscope and ports for accessories such as a video processing apparatus or the suction device, for example in the form of a vacuum pump. The handle is preferably designed in such a way that it can be ergonomically gripped and operated by the user or operator or surgeon with just one hand. This means that all important operating elements on the handle are preferably arranged in such a way that the user can operate them with one hand without having to change a position of his or her hand. The handle may comprise a shell. Preferably, the shell is made from a plastic material. Furthermore, the shell preferably comprises two half-shells, a first half-shell and a second half-shell which are connectable with each other in a separation plane or dividing plane or split plane. In case of a provision of a proximal endoscope interface, said endoscope interface may be prepared or provided to be connectable to e.g. a robotic arm.

The endoscope further comprises the insertion cord, preferably having a distal tip unit formed at the distal end of the insertion cord. The distal tip unit may comprise an image capturing means like a camera and a light emitting device like a LED.

Furthermore, the endoscope comprises the working channel. The working channel is provided and configured to transport fluid, mucus and tissue parts or particles from the patient's body cavity. Furthermore, the working channel may be configured to guide a surgical tool or instrument through it to a surgical location in the patient's body. For this purpose, the working channel may have a Y-connector in the handle that allows insertion of the surgical tool or instrument into the working channel.

The endoscope further comprises the suction valve arranged in the endoscope handle or interface.

The suction valve comprises the valve body, which may also be designated as a valve housing. The valve body may be formed cylindrically at least in portions and may thus be described as including a valve cylinder that extends in an axial direction of the suction valve.

The inlet and the outlet of the suction valve are preferably formed as integral parts or portions of the valve body. The inlet and the outlet may have substantially a geometry of a pipe section, preferably of a pipe section with an annular cross-section.

The inlet and outlet may extend away from an outer cylindrical surface of the valve cylinder of the valve body.

The inlet is provided and configured to be connected to the working channel. The inlet may be connected directly to the working channel. Alternatively, the inlet may be connected to the working channel via a suction channel, especially preferably to the Y-connector of the working channel. The suction channel may e.g. be configured as a separate hose or tube.

The outlet is provided and configured to be connected to the suction device. The outlet may be connected directly to the suction device. Alternatively, a hose may be formed between the outlet and the suction device. Alternatively or additionally, at least one coupling element and/or at least one deflector element and/or at least one pipe element may be formed between the outlet and the suction device.

The suction valve further comprises the stem. The stem is arranged in the valve body in such a way that the stem is able to move in the axial direction of the suction valve in the valve body, especially in the valve cylinder of the valve body. The axial movement, at least in one direction, can be caused by pushing a button. The button may be a button portion which is preferably formed integrally with preferably a remainder of the stem at an end portion of the stem. Alternatively, the button may be a button element attached to the stem, in particular to the end portion of the stem. The button portion or element may protrude axially from the valve body, so that a user can press the button portion or element in order to move the stem axially in the valve body. The stem can be moved in the valve body in such a way that it shifts the suction valve at least between the valve closed state of the suction valve, in other words in its first position, and the valve open state of the suction valve, in other words in its second position. In other words, the stem may be configured to seal between an inlet opening and an outlet opening in the suction valve closed state, and the stem may be configured to enable a fluid flow between the inlet opening and the outlet opening of the valve body in the suction valve open state. In still other words, the suction valve may be configured to manually control the suction through the working channel, in particular by the user pressing the button towards the housing of the endoscope. In this way, the user can easily control the suction valve and can switch between the valve closed state and the valve open state.

The suction valve is mounted between the first half-shell and the second half-shell. In other words, the suction valve is arranged in the dividing plane of the handle and is fixed to the first half-shell and/ or the second half-shell.

The suction valve may be locked in the first half-shell and/ or the second half-shell.

The first half-shell comprises the first fixing portion and the second fixing portion. The first fixing portion is positioned further outwardly than the second fixing portion. In other words, the first fixing portion is positioned closer to a circumferential or peripheral edge of the shell, which is positioned in the dividing plane, than the second fixing portion.

The first fixing portion is provided and configured to receive the first portion of the suction valve. "Receiving" means that the first portion is held by the first fixing portion, for example in a form fitting manner. Alternatively or additionally, a frictional connection such as press fit may be provided.

The second fixing portion is provided and configured to receive the second portion of the suction valve. "Receiving" means that the second portion is held by the second fixing portion, for example in a form fitting manner. Alternatively or additionally, a frictional connection may be provided.

The first portion of the suction valve is different from the second portion of the suction valve. In other words, the first portion is spaced from the second portion.

Preferably, the first portion and the second portion of the suction valve may be formed of different materials.

Further preferably, the first portion and the second portion may have substantially different geometric shapes. For example, the first portion may have a round geometry and the second portion may have an angular geometry.

Such a configuration of the endoscope allows the suction valve to be positioned and fixed in the handle easily and without the use of tools. The formation of two different mounting points between the suction valve and the first half-shell ensures that the suction valve is firmly fixed in the handle without twisting. Furthermore, such a configuration can prevent incorrect assembly, for example by the poka yoke principle.

The suction valve, in particular the parts thereof, may be made of a plastic material or polymer. Especially preferred, the suction valve, in particular parts thereof, preferably the valve body and the stem, may be manufactured in an injection molding process, i.e. may be injection molded parts.

The second half-shell may comprise a third, outer fixing portion and a fourth, inner fixing portion. The third, outer fixing portion may be configured to receive the first portion of the suction valve, and the fourth, inner fixing portion may be configured to receive the second portion of the suction valve.

The third fixing portion may be provided and configured to receive the first portion of the suction valve. "Receiving" means that the first portion is held by the third fixing portion, for example in a form fitting manner. Alternatively or additionally, a frictional connection may be provided. The third fixing portion may be configured to suppress a degree of freedom of the suction valve, in particular at least partially or in one direction.

The fourth fixing portion may be provided and configured to receive the second portion of the suction valve. "Receiving" means that the second portion is held by the fourth fixing portion, for example in a form fitting manner. Alternatively or additionally, a frictional connection may be provided. The fourth fixing portion may be configured to suppress a degree of freedom of the suction valve, in particular at least partially or in one direction.

Since the first half-shell and the second half-shell are mounted to each other to form the handle, it is advantageous if the third fixing portion and the fourth fixing portion, which also secure the suction valve, are formed on the second half-shell. In this way, the mounting of the suction valve can be further improved and a more rigid connection between the first half-shell, the second half-shell and the suction valve can be achieved.

In another aspect, the first outer fixing portion of the first half-shell and the third outer fixing portion of the second half-shell may be formed identically or symmetrically to each other.

In other words, the third fixing portion of the second half-shell may correspond to the first fixing portion of the first half-shell, and/ or the third fixing portion of the second half-shell may correspond to the first fixing portion of the first half-shell mirrored on the split plane or dividing plane of the handle.

Such a matched configuration of the first fixing portion and the third fixing portion ensures uniform force transmission between the suction valve, the first half-shell and the second half-shell. This is particularly advantageous because the first fixing portion and the third fixing portion are each the outer fixing portions, which are close to a force application point of the suction valve.

In another aspect, the first, outer fixing portion of the first half-shell and the third, outer fixing portion of the second half-shell may preferably substantially completely encircle the first portion of the suction valve in a circumferential direction.

The first fixing portion and the third fixing portion may each have a semi-circular shape which, in the assembled state, that is, in the state when the first half-shell is assembled to the second half-shell and thus the housing or handle shell is formed, form a circular shape which completely encloses the first portion of the suction valve, which may be circular in shape.

The first fixing portion and the third fixing portion may form a collar shape that encloses the first portion of the suction valve.

Such a configuration of the first fixing portion and the third fixing portion can, in addition to further improving the force transmission, implement a seal between the suction valve and the handle. In particular, the sealing can be implemented without additional sealing elements, which further reduces the cost of the endoscope and makes the endoscope easier and cheaper to assemble.

In another aspect, the first fixing portion and/ or the third fixing portion may be formed as a portion of the handle shell, preferably of the half shell, having a groove or a recess.

The second, inner fixing portion of the first half-shell and the fourth inner fixing portion of the second half-shell may be formed differently.

The second fixing portion and the fourth fixing portion may have different geometries of different shapes or, they may differ more or further than just being mirrored. For example, the second fixing portion may have a cylindrical shape or comprise a cylinder and the fourth fixing portion then may not have that cylindrical shape or comprise such a cylinder but e.g. have a rectangular shape. The second fixing portion may comprise shapes or shaped portions, that the fourth fixing portion does not comprise.

Preferably, the mounting in the second fixing portion may differ from the mounting in the fourth fixing portion. Alternatively or additionally, a clearance of the second fixing portion may differ from a clearance of the fourth fixing portion.

Different fixations in the second fixing portion and the fourth fixing portion can facilitate an assembly process of the handle. Furthermore, excessive tensioning of the suction valve between the second fixing portion of the first half-shell and the fourth fixing portion of the second half-shell can be prevented by the different fixations.

In another aspect, the second, inner fixing portion of the first half-shell and the fourth inner fixing portion of the second half-shell may be configured as tower geometries protruding from outer shell portions or shell skins of the half-shells into an interior space of the endoscope handle or interface.

The tower geometries make it possible to hold the suction valve in the center of the handle without having to make the corresponding portion on the suction valve too long, which could reduce the stability of the portion.

As described above, the second, inner fixing portion may be configured to suppress at least one degree of freedom of the suction valve and the fourth, inner fixing portion may be configured to suppress a remaining degree or remaining degrees of freedom of the suction valve, so that the suction valve is fixed in its position and orientation in the housing of the endoscope handle or interface. It is to be understood that this preferably means that it is prevented that the suction valve may change its position or orientation in the endoscope housing. It is to be understood that the second, inner fixing portion may be configured to suppress at least one degree of freedom of the suction valve and the fourth, inner fixing portion is configured to suppress a remaining degree or remaining degrees of freedom of the suction valve, but that does not necessarily mean that the second, inner fixing portion and the fourth, inner fixing portion are together configured to suppress all degrees of freedom of the suction valve by themselves, i.e. alone, this is only preferred. As described above, the first and/or third, outer fixing portions may also suppress a degree of freedom of the suction valve, in particular at least partially or in one direction, therefore it is not necessary that this degree of freedom is then also suppressed by the second, inner fixing portion or the fourth, inner fixing portion. In other words, the fourth, inner fixing portion may be configured to suppress a degree or degrees of freedom that are not suppressed by the first, second or third fixing portions.

Preferably, the second, inner fixing portion and the fourth, inner fixing portion are configured to suppress together all degrees of freedom of the suction valve, in particular three positional and three rotational degrees of freedom. Preferably the second, inner fixing portion and the fourth, inner fixing portion are configured to fixate together the suction valve in position and orientation.

Preferably, the second, inner fixing portion is configured to suppress more degrees of freedom of the suction valve than the fourth, inner fixing portion.

Preferably, the second, inner fixing portion is configured to suppress (due to its shape) translational movements except in a direction towards the second half-shell and/or to the fourth fixing portion, when the suction valve is inserted, i.e. in the assembled state of the endoscope. Preferably, the fourth, inner fixing portion (of the second half-shell) is configured to prevent/suppress this movement.

Preferably, the second, inner fixing portion is configured to only allow a translational movement of the suction valve in direction of or to the second half-shell, when the suction valve is inserted, i.e. in the assembled state of the endoscope. In other words, preferably the second, inner fixing portion is configured to suppress by its shape arbitrary rotations and all translations but one translational movement. It is to be understood that the second, inner fixing portion may nevertheless provide some small rotational looseness by its shape making it easier to insert a corresponding portion of the suction valve.

Preferably, the fourth, inner fixing portion of the second half-shell is configured to prevent or suppress this remaining one translational movement. This makes an assembly easier and allows critical tolerances for fixation of the suction valve to be limited to the first half-shell.

Preferably, the second, inner fixing portion of the first half-shell has a receptacle configured to hold or receive a portion of the suction valve, such as a fixing projection of the suction valve.

In the following, the receptacle of the second, inner fixing portion is described:
Preferably, the receptacle of the second, inner fixing portion has substantially a polygonal shape or cross-section. More preferably, it has a substantially rectangular cross-section and is therefore slot-shaped.

Preferably, the rectangular cross-section tapers slightly. A rectangular cross-section is to be understood as a cross-section, which can hold or receive another body of rectangular shape. It may comprise a split slot and/or be made of or comprise two separate portions.

Preferably, the second, inner fixing portion is made of or comprises two separate portions or brackets spaced from each other.

Preferably, these two portions are both formed as first tower geometries, protruding from the first half-shell in direction towards the centre of the housing and/or the second half-shell.

Preferably, these two portions are mirrored portions. Preferably, one of these portions may result by mirroring the other one at a mirror plane between them.

Preferably, the two portions are U-shaped brackets opposed to each other forming the slot-shaped receptacle. U-shaped is to be understood as being able to cover something inside from three sides, at least partially. Preferably, the two U-shaped brackets form a split slot in between them. Such a split slot can add flexibility and save material.

The split slot is preferably formed in between the first tower geometries or in other words in between two portions of the second, inner fixing portion. A first tower geometry may be understood as a geometry, which extends preferably perpendicularly away from an outer shell portion of the first half-shell in the direction of the interior of the handle. Preferably, the extension towards the interior is greater than an extension parallel to the outer shell portion or shell skin.

Preferably, the receptacle may taper slightly.

More preferably, the receptacle is reduced linearly and/ or continuously going further inside, in direction from the center of the housing to an outer shell portion of the first half-shell. In other words, the receptacle may have an angled interface, e.g. a drafting angle.

Preferably, the receptacle has at least one angled interface or wall or inner surface portion also serving as an inlet chamfer.

Therefore, the suction valve or a portion of the suction valve may be press-fitted inside. In other words, the receptacle may be configured for a press-fit with a corresponding portion of the suction valve.

Preferably, the receptacle tapers slightly in two directions or dimensions, in particular in width and height, wherein the third axis going inside the receptacle(s) may be described as length. Preferably, the cross-section of the receptacle is rectangular, having a width and a height. Especially in this case, the two sides opposite to each other, respectively, may get closer to each other further inside the receptacle. In other words, the width and height of the receptacle may reduce.

Additionally or alternatively, the receptacle may be of constant width and/or height and/or configured for a form-fit with a corresponding portion of the suction valve. In other words, the taper of the receptacle of the second, inner fixing portion may be small so that the second, inner fixing portion of the first half-shell may be defined as comprising a slot of essentially constant width, despite of the small taper. It may also be provided that the slot is exactly of constant width, i.e. without taper.

Preferably, the fourth, inner fixing portion of the second half-shell may comprise a receptacle configured to hold or receive a portion of the suction valve, such as a fixing projection of the suction valve.

In the following, the receptacle of the fourth, inner fixing portion is described:
Preferably, the receptacle of the fourth, inner fixing portion is V-shaped and thereby configured to guide the suction valve or a portion of the suction valve into it, in particular during assembly.

Preferably, the V-shaped receptacle guides, during assembly, the suction valve into it in a, preferably the only, direction of movement of the suction valve that is not suppressed by the shape of the second, inner fixing portion. In other words, preferably the V-shaped receptacle guides the suction valve during assembly into it in a direction, wherein the second, inner fixing portion does not suppress movement of the suction valve in that direction by its shape. This makes the assembly much easier.

It is to be understood, that the term V-shaped does not mean that it necessarily has completely the shape of a "V", but substantially and therefore guiding the suction valve inside this V-shaped receptacle and making the assembly easier. In other words, V-shaped is not to be understood as purely geometrical but more as functional.

Preferably, the V-shaped receptacle of the fourth, inner fixing portion may comprise a force-taking surface portion as an inner surface of the receptacle that is provided to be in contact with the suction valve. Preferably, this force-taking surface portion is planar or flat and preferably perpendicular to a direction of the axis of the stem and/or a direction into which the button may be pressed and/or located in the V-shaped receptacle on a side opposite to the stem and/or button or on a side opposite to the direction in which the button is configured to be pressed. Thereby, when the button is pressed, this force-taking surface portion may take the pressing force from the suction valve and provide stability. Further, it may prevent a rotational and/or a translational movement of the suction valve due to the pressing force.

The force-taking surface portion may be perpendicular to a direction in which the stem is axially movable and/or the button may be pushed, and/or may be located on an inner side of the V-shaped receptacle opposite to the stem and/or button.

The, preferably flat, force-taking surface portion may be perpendicular to a direction in which the button of the suction valve is pushable to move the stem, so that if or when the button is pushed, a movement of the suction valve caused by the pushing of the button in that direction relative to the housing is suppressed by the flat force-taking surface portion.

Preferably, the V-shaped receptacle may comprise a planar or flat end surface portion as end or landing or docking surface of the receptacle. Preferably, this is an inner portion being the furthest inside the V-shaped receptacle, thereby flattening the V-shaped receptacle so that it is not pointy. Preferably, this end surface portion is provided and/or configured so that the suction valve or a portion of the suction valve may be adjacent to it or abut it flatly, when the endoscope is assembled.

The V-shaped receptacle of the fourth, inner fixing portion may preferably be formed on a second tower geometry. A second tower geometry may be understood to be a geometry, which extends substantially perpendicularly away from an outer shell portion or shell skin of the second half-shell in the direction of the interior of the handle. Preferably, the extension towards the interior is greater than an extension parallel to the outer shell portion or shell skin.

Preferably, the fourth, inner fixing portion is made of or comprises two separate portions or brackets spaced from each other.

Preferably, these two brackets are both second tower geometries protruding from the second half-shell in direction towards the centre of the housing and/or the first half-shell.

Preferably, both these two brackets are shaped identical and/or facing each other.

Such a configuration of the second fixing portion and the fourth fixing portion can further simplify the assembly of the handle. The second substantially slot-shaped fixing portion in the first half-shell can firmly position the suction valve, and the V-shape of the fourth fixing portion can significantly simplify the process of introducing the portion of the suction valve into the fourth fixing portion, which is particularly advantageous when assembling the first half-shell with the second half-shell, where a view of the fourth fixing portion is obscured by the second half-shell.

In another aspect, the first portion of the suction valve may be the valve spring.

The first fixing portion of the first half-shell and/ or the third fixing portion of the second half-shell may contact and hold the valve spring of the suction valve, preferably circumferentially.

The valve spring may preferably be formed by an elastic material, in particular an elastomer.

By fixing the suction valve via the valve spring, a reliable sealing between the handle and the suction valve can be achieved. This sealing can be further increased if the valve spring is made of an elastic material, in particular an elastomer. In addition, the formation of further fastening geometries on the suction valve can be omitted, which reduces the number of components or the complexity of the components and thus the costs and assembly effort for the suction valve and the endoscope.

In another aspect, the second portion of the suction valve may be at least one projection formed integrally with the valve body.

The second portion may be an integral section of the valve body, which is injection molded together with the valve body. The second portion may include at least one projection, preferably two projections.

In another aspect, the second portion may comprise a first projection formed integrally with the valve body and a second projection formed integrally with the valve body, said first projection and said second projection may project in opposite directions from said valve body, in particular from the valve cylinder of the valve body.

The second portion may comprise the two wing-shaped projections formed radially opposite each other on the valve body. The first projection and the second projection may be symmetrical or identical. Alternatively, it is conceivable to form the first projection and the second projection differently, for example to implement the poka yoke principle. The first projection and the second projection may differ in width, length, thickness and/or cross-sectional geometry.

In another aspect, the second portion of the suction valve may comprise a stop, which defines a maximum penetration depth of the second portion.

The first projection and/ or the second projection may comprise a stop. The stop may be in the form of a reinforcing rib or in the form of an annular rib or in the form of shoulders formed on the projection or the like.

Such a stop can simplify the assembly of the handle, reducing assembly time and thus assembly costs, since an assembler does not have to align the projection manually but simply pushes it in until the stop prevents further insertion.

In another aspect, the first half-shell may comprise retention hooks, which are formed directly adjacent to at least the first, outer fixing portion to close the housing.

Latching geometries in the form of retention hooks may be formed between the first half-shell and the second half-shell to allow the handle to be closed or assembled without tools. In particular, the latching geometries may be formed directly adjacent to the first fixing portion to increase a clamping force on the first fixing portion and/ or the third fixing portion to improve sealing and fixation of the suction valve in the handle.

In another aspect, the valve body and/ or the stem are preferably made from a thermoplastic polymer, e.g. polypropylene (PP) especially biodegradable polypropylene, polystyrene (PS), polycarbonate (PC), acrylonitrile butadiene styrene (ABS), polyoxymethylene (POM), etc. However, also other polymer materials or other materials are conceivable.

According to another aspect, which can be possibly independently claimed, the present disclosure further relates to an endoscope that comprises a proximal endoscope handle or interface, an insertion cord extending from the endoscope handle or interface and configured to be inserted into a patient's body cavity and a working channel configured to let fluid and mucus sucked from the patient's body cavity flow therethrough. The endoscope handle or interface comprises a housing and a suction valve configured to control a suction through the working channel and having a valve closed state and a valve open state. The housing comprises a first half-shell and a second half-shell The suction valve comprises a valve body having an inlet connected to the working channel, an outlet provided and configured to be connected to a suction device and a stem movable axially in the valve body between at least a first position, in which the suction valve is in the valve closed state, and a second position, in which the suction valve is in the valve open state. The suction valve is mounted between the first half-shell and the second half-shell. The first half-shell comprises a first, outer fixing portion and a second, inner fixing portion. The first, outer fixing portion is configured to receive a first portion of the suction valve and the second, inner fixing portion is configured to receive a second portion of the suction valve. The second half-shell comprises a third, outer fixing portion and a fourth, inner fixing portion. The third, outer fixing portion is configured to receive the first portion of the suction valve, and the fourth, inner fixing portion is configured to receive the second portion of the suction valve. The second, inner fixing portion of the first half-shell and the fourth inner fixing portion of the second half-shell have a different geometry. The second, inner fixing portion of the first half-shell comprises a slot, preferably of constant width, and the fourth inner fixing portion of the second half-shell comprises a(n essentially) V-shaped slot.

The object of the present disclosure is further solved by a method of assembling an endoscope as described above, comprising the steps:
- Sliding a valve spring onto a valve body of a suction valve and fixing or latching the valve spring to a stem of the suction valve;
- Positioning the suction valve in a first half-shell of a housing of an endoscope handle or interface by inserting a second portion of the suction valve in a second, inner fixing portion of the first half-shell of the housing;
- Inserting a first portion of the suction valve in a first, outer fixing portion of the first half-shell;
- Positioning a second half-shell of the housing on the first half-shell of the housing;
- Inserting the second portion of the suction valve in the fourth, inner fixing portion of the second half-shell of the housing;
- Inserting the first portion of the suction valve in a third, outer fixing portion of the second half-shell;
- Closing the half-shells.

In other words, the object of the present disclosure is also solved by the method of assembling or mounting the endoscope according to any of the above aspects.

In a first step, the valve spring is fitted onto the valve body so that the valve body and valve spring are connected to each other. The connection between the valve spring and the valve body can be a force-fit and/or a form-fit connection.

In a second step, the suction valve is positioned in the first half-shell, with a first projection of the second portion of the valve body being inserted, preferably as far as the stop allows, into the second fixing portion.

In a third step, the first portion of the suction valve, which is preferably the valve spring, is inserted into the first fixing portion of the first half-shell, which preferably comprises a groove.

In a fourth step, the two half-shells are positioned relative to each other so that their openings are facing each other.

In a fifth step, the two half-shells are joined together, with the second projection of the second portion of the suction valve being inserted into the fourth fixing portion. The fourth fixing portion preferably includes the V-shape to facilitate insertion.

In a sixth step, the first portion of the suction valve, which is preferably the valve spring, is inserted into the third fixing portion of the second half-shell, which preferably comprises a groove.

In a seventh step, the first half-shell and the second half-shell are closed to form the handle of the endoscope. Preferably, latching hooks formed between the first half-shell and the second half-shell are engaged with each other. In other words, the first half-shell and the second half-shell are attached and/ or assembled to each other.

The present disclosure further relates to a system comprising the endoscope according to one of the above aspects and a video processing apparatus (VPA) couplable to the endoscope and capable of outputting a live image recorded by an image sensor of the endoscope on a display. The video processing apparatus may comprise a built-in display and/ or a detachable display and/or may be couplable to an external display.

The video processing apparatus may be a video processing apparatus showing an image or a video captured by an image capturing means arranged at the distal tip unit.

Furthermore, the present disclosure relates to a system, that may also be claimed independently, comprising the endoscope described above wherein the system further comprises a suction device that is connectable directly or indirectly, for example through a hose, to the outlet of the valve body of the suction valve.

The suction device may be a vacuum pump or the like. There are also embodiments imaginable, in which the suction device is directly coupled to the endoscope or is part of the endoscope.

### Brief description of the figures

The disclosure is explained in more detail below using preferred embodiments and referring to the accompanying figures.
Fig. 1 shows a side view of an endoscope according to the present disclosure.
Fig. 2 shows a first half-shell of the endoscope handle or interface having a suction valve.
Fig. 3 shows the suction valve mounted in the first half-shell in an enlarged view.
Fig. 4 shows the first half-shell in a perspective view.
Fig. 5 shows a first enlarged view of the first half-shell.
Fig. 6 shows a second enlarged view of the first half-shell.
Fig. 7 shows a first enlarged view of a second half-shell.
Fig. 8 shows a second enlarged view of the second half-shell.
Fig. 9 shows a sectional view of the first half-shell and the second half-shell mounted to each other.
Fig. 10 shows a schematic view of the suction valve mounted in the handle.
Fig. 11 shows a sectional view of the suction valve mounted in the handle.
Fig. 12 shows a perspective view of the suction valve mounted in the first half-shell.
Fig. 13 shows a sectional view of the suction valve mounted between the first half-shell and the second half-shell.
Fig. 14 shows a preferred embodiment of a fourth fixing portion according to the present disclosure.
Fig. 15 shows the preferred embodiment of the fourth fixing portion of Fig. 14 enlarged.
Fig. 16 shows a perspective view of a preferred embodiment of a second fixing portion according to the present disclosure.
Fig. 17 shows a sectional view of the preferred embodiment of the second fixing portion of Fig. 16.
Fig. 18 shows another sectional view of the preferred embodiment of the second fixing portion of Fig. 16.

The figures are schematic in nature and serve only to understand the disclosure. The features of the different embodiments can be interchanged among each other.

### Detailed description of the figures

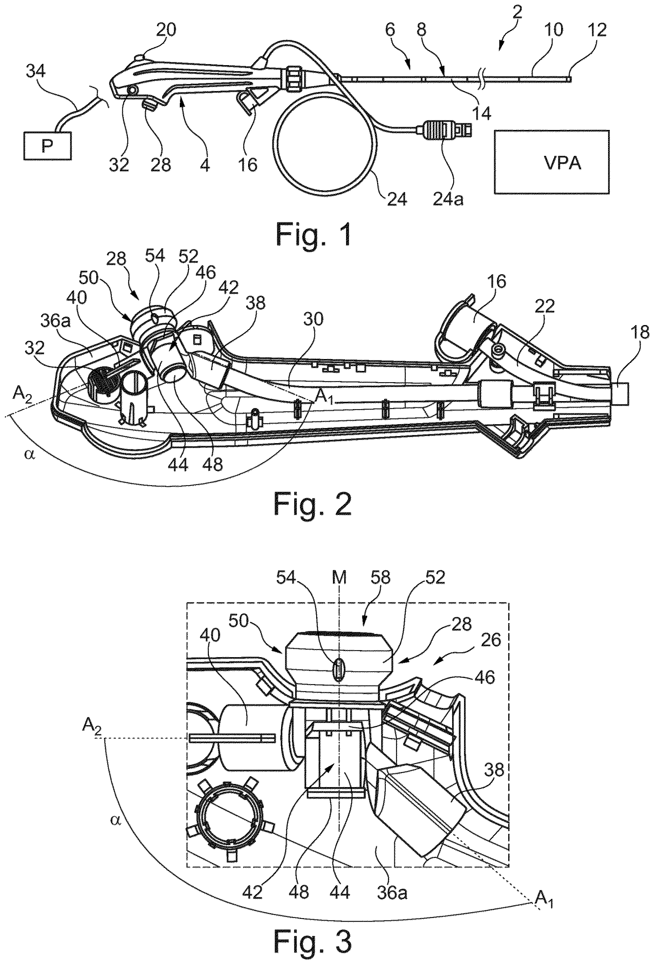

Fig. 1 shows a side view of an endoscope 2 according to the present disclosure, which is preferably a single-use endoscope. The endoscope 2 comprises: a proximal endoscope handle 4 and an insertion cord 6 extending distally from the endoscope handle 4. The insertion cord 6 is configured to be inserted into a patient's body cavity and comprises an insertion tube 8, a bending section 10 and a distal tip unit 12. The endoscope 2 further comprises a working channel 14 which extends from a biopsy port or working channel access port 16 provided at the endoscope handle 4 to the distal tip unit 12. The working channel 14 comprises a working channel tube 18 (shown in Fig. 2), which is arranged inside the endoscope handle 4, the insertion tube 8, and the bending section 10. The endoscope handle 4 comprises an operating unit 20, formed as a lever, for bending the bending section 10 of the insertion cord 6. The working channel tube 18 and the working channel access port 16 are connected via a Y-connector 22. The endoscope 2 is connectable to a video processing apparatus VPA, which is schematically illustrated via a connecting cable 24 comprising an electrical connector 24a insertable in a socket (not shown) of the video processing apparatus VPA. Alternatively the endoscope 2 may be wirelessly connectable to the video processing apparatus VPA, e.g. by a wireless HDMI connection.

When a surgical instrument or tool is inserted into the working channel 14, the surgical instrument or tool is inserted into the working channel access port 16 and guided through the Y-connector 22 into the working channel tube 18.

The endoscope 2 further comprises a suction valve 26, having a suction button 28 formed on the handle 4 and configured to control a suction power through the working channel 14 of the endoscope 2. The suction valve 26 is connected to a suction channel 30 and a vacuum port 32, which protrudes from or is embedded in the handle 4 (shown in Fig. 2). The endoscope 2 is connectable to a suction pump P, which is schematically illustrated in Fig. 1, via a hose 34, which is connectable to the vacuum port 32 of the endoscope 2.

Fig. 2 shows the handle 4 in an open state. Specifically, the handle 4 is formed essentially from two half-shells 36, a first half-shell 36a and a second half-shell 36b, that form the housing of the endoscope handle 4. In Fig. 2, the second half-shell 36b is removed and all other elements of the endoscope 2, which are not necessary for an understanding of the present disclosure, are also not shown in Fig. 2 for the sake of clarity and comprehensibility of the disclosure. It is to be understood that all other mechanical, optical and electronic components, which are formed in the handle 4 in a conventional endoscope, are or may be formed in the endoscope 2 of the present disclosure.

The suction channel 30 connects the suction valve 26, which is mounted between the first half-shell 36a and the second half-shell 36b at a proximal end portion of the handle 4, to the Y-connector 22 and runs in a longitudinal direction of the handle 4, that is, essentially in a direction, which continues the insertion cord 6. The suction valve 26 comprises an inlet 38, which is configured to connect to the suction channel 30. In other words, the inlet 38 is configured as a connector, which connects to the suction channel 30. The suction channel 30 has a tubular shape and may be in the form of an elastomeric tube or a rigid pipe. The suction valve 26 further comprises an outlet 40, which is connectable to the vacuum port 32. In other words, the outlet 40 is configured as a connector, which connects to the vacuum port 32.

In the following, the external structure of the suction valve 26 is explained in more detail with reference to Figs. 2 and 3. Fig. 3 is an enlarged view of the suction valve 26 in the assembled state, whereby the suction channel 30 and the vacuum port 32 are not shown in Fig. 3.

The suction valve 26 comprises a valve body 42, which comprises a valve cylinder 44 extending in an axial direction of the suction valve 26. The valve body 42 further comprises the inlet 38 and the outlet 40. The inlet 38 and the outlet 40 each have a substantially tubular geometry with a substantially circular cross-sectional configuration. Seen in the side view of the endoscope handle 4 in an assembled state of the suction valve in Fig. 3, the inlet 38 has a first center axis A1 and the outlet 40 has a second center axis A2. The first center axis A1 extends in a center position of the inlet 38 in a direction of extension of the inlet 38. The second center axis A2 extends in a center position of the outlet 40 in a direction of extension of the outlet 40. An angle α is enclosed between the first center axis A1 and the second center axis A2. The angle α is greater than 90° and less than 180°. Preferably, the angle is greater than 110°. Particularly preferably, the angle is about 120°.

The valve body 42 is formed in one piece from a plastic material, preferably polypropylene. Further, the valve body 42 comprises fixing projections 46 formed thereon, which are configured to fix the suction valve 26 in the handle 4 between the half-shells 36a and 36b. Specifically, the suction valve 26 includes two fixing projections 46 which extend oppositely radially outwardly away from the valve cylinder 44. The fixing projection 46 will be described in more detail below.

The suction valve 26 further includes an end cap 48 formed on an end portion of the valve cylinder 44 facing an interior of the handle 4, and closing the valve cylinder 44. Specifically, the end cap 48 closes a substantially circular bottom opening of the valve body 42. The end cap 48 and the valve body 42 are welded together. Alternatively, it is conceivable that the end cap 48 may be fixed in place by adhesive bonding or by a latching action.

A valve spring 50 is formed on an end portion of the valve cylinder 44 opposite the end cap 48. The valve spring 50 protrudes for the most part from the housing of the handle 4 and ensures that the suction valve 26 is maintained in an initial valve closed state or is returned to the initial valve closed state without a user actuation. A bypass hole 54 is formed in a side surface 52 of the valve spring 50. The bypass hole 54 allows ambient air to be drawn through the suction valve 26 into the outlet 40 in a closed state of the suction valve, that is, in a state in which the volume flow from inlet 38 to outlet 40 is blocked.

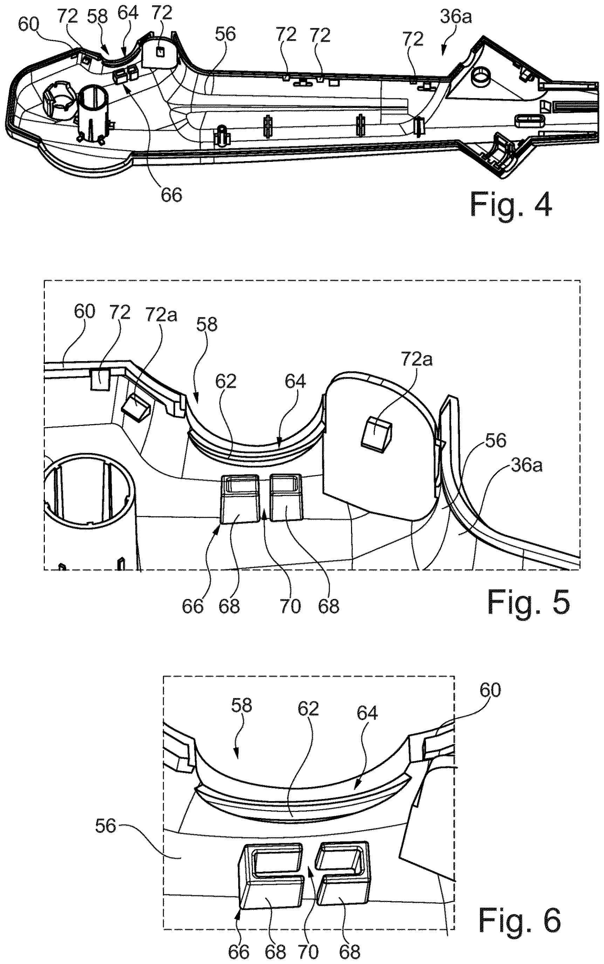

Fig. 4 shows the first half-shell 36a. Fig. 5 shows an enlarged view of the first half-shell 36a in an area of the first half-shell 36a in which the suction valve 26 is received. Fig. 6 shows a further enlarged view of receiving geometries in said area. In the following, the receiving geometries for the suction valve 26 in the first half-shell 36a are described in more detail with reference to Figs. 4, 5 and 6.

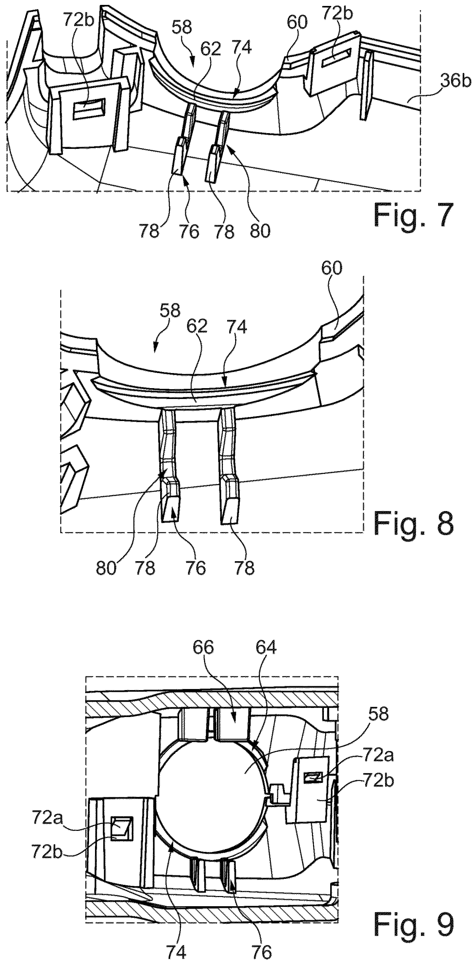

The first half-shell 36a comprises a shell skin 56 forming an outer wall of the handle 4. A recess 58 is formed in the shell skin 56, the recess 58 being configured to allow the suction valve 26 to extend outwardly from the interior of the handle 4 through the shell skin 56 of the handle 4. The recess 58 has a shape substantially in the form of a semicircle and extends from a contact edge 60 at which the first half-shell 36a and the second half-shell 36b abut one another in an assembled state of the handle 4. Specifically, the recess 58 of the first half-shell 36a is configured to form a circular opening in the shell skin 56 of the handle 4 in conjunction with the recess 58 of the second half-shell 36b when the first half-shell 36a and the second half-shell 36b are assembled (shown in Fig. 9).

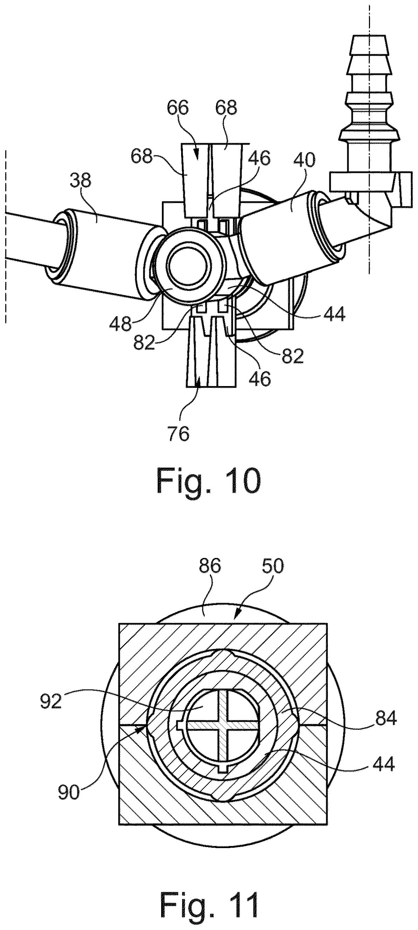

Directly adjacent to the recess 58, towards an interior space enclosed by the handle 4, a groove 62 is formed which, in conjunction with the recess 58, forms a first outer fixing portion 64 according to the preferred embodiment. The groove 62 extends into the shell skin 56, starting from the contact edge 60 in the area of the recess 58. The groove 62 may extend completely around the recess 58 or around a portion of the recess 58. The function of the first fixing portion 64 is described in more detail below with reference to Figures 10 to 13.

Parallel to the groove 62, offset towards the interior space of the handle 4, that is, further away from the recess 58, a second inner fixing portion 66 extends, starting from the shell skin 56, perpendicularly into the interior space. The second fixing portion 66 comprises two U-shaped brackets 68 extending away from the shell skin 56 facing each other and forming a substantially rectangular or slot-shaped receptacle 70. The rectangular receptacle 70 is oriented parallel to the groove 62 and forms a cuboid-shaped receiving space. The two U-shaped brackets 68 face each other with arm sections of the U-shape. The two U-shaped brackets 68 extend from the shell skin 56 in a tower shape. The function of the second fixing portion 66 is described in more detail below with reference to Figures 10 to 13.

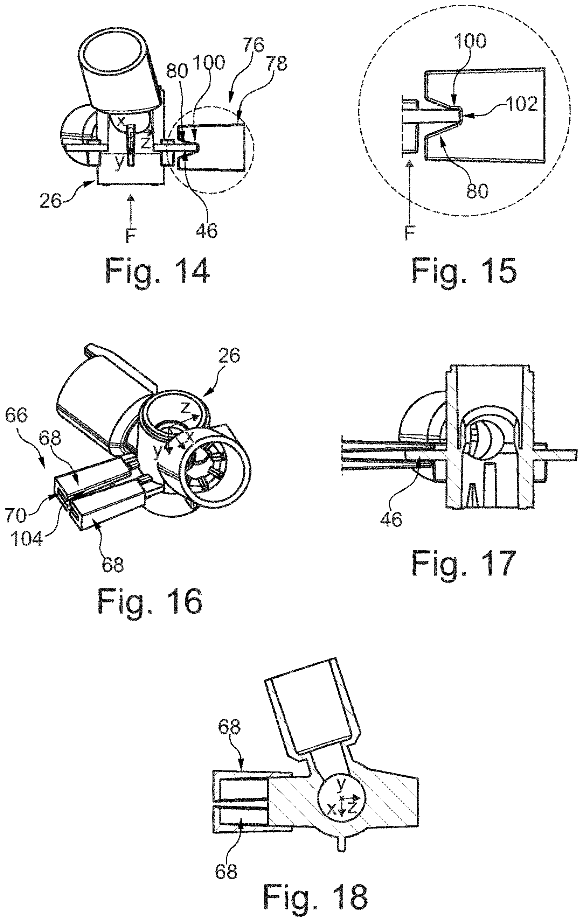

Embodiments are also conceivable, in which the receptacle 70 slightly tapers toward the shell skin 56 as shown in Figures 16 to 18.

Furthermore, embodiments are conceivable which are not formed from two U-shaped brackets 68 but from a single, preferably rectangular element having the rectangular or slot-shaped receptacle 70.

The first half-shell 36a is a one-piece component injection molded from a plastic, preferably a thermoplastic.

Adjacent to the recess 58, retention hooks 72a are formed on the first half-shell 36a on the shell skin 56, which are configured to latch into corresponding counterparts 72b (shown in Fig. 7) in the second half-shell 36b to connect the first half-shell 36a and the second half-shell 36b together. Such retention hooks 72a are further evenly distributed around the entire circumference of the first half-shell 36a, preferably along the contact edge 60.

Fig. 7 and Fig. 8 show the receiving geometries for the suction valve 26 in the second half-shell 36b. Since the second half-shell 36b corresponds in large parts to the first half-shell 36a, only differences between the second half-shell 36b and the first half-shell 36a will be described in the following.

Directly adjacent to the recess 58 of the second half-shell 36b, towards an interior space enclosed by the handle 4, the groove 62 is formed which, in conjunction with the recess 58, forms a third outer fixing portion 64. The groove 62 extends into the shell skin 56, starting from the contact edge 60 in the area of the recess 58.

Thus, the third fixing portion 74 of the second half-shell 36b corresponds to the first fixing portion 64 of the first half-shell 36a. In other words, the first fixing portion 64 and the third fixing portion 74 are identical in configuration. In still other words, the first fixing portion 64 and the third fixing portion 74 are symmetrical to each other with respect to a splitting layer or dividing plane of the handle 4. The splitting layer is a layer, which contains the contact edge 60.

Parallel to the groove 62, offset towards the interior space of the handle 4, that is, further away from the recess 58, a fourth inner fixing portion 76 extends, starting from the shell skin 56, perpendicularly into the interior space of the handle 4. The fourth fixing portion 76 comprises two V-shaped brackets 78 extending away from the shell skin 56 facing each other and forming a substantially V-shaped receptacle 80. The V-shaped receptacle 80 tapers from the interior space of the handle 4 to the shell skin 56. The V-shaped receptacle 80 is oriented parallel to the groove 62 and forms a three-sided straight prism-shaped receiving space. In other words, a receiving space that tapers toward the shell skin 56. The two V-shaped brackets 78 are each formed in a plate shape and parallel to each other. The two V-shaped brackets 78 extend from the shell skin 56 in a tower shape. The function of the second fixing portion 66 is described in more detail below with reference to Figures 10 to 13.

Furthermore, embodiments are conceivable which are not formed from two V-shaped brackets 78 but from a single, preferably rectangular element having the three-sided straight prism-shaped receiving space shaped receptacle 80.

In one embodiment, a portion of the V-shaped receptacle 80 that is formed closest to the shell skin 56 may include a parallel slot-shaped portion. In other words, the V-shaped receptacle 80 can change over its course into a regular rectangular slot shape.

The second half-shell 36b is a one-piece component injection molded from a plastic, preferably a thermoplastic.

Adjacent to the recess 58, the counterparts 72b are formed on the second half-shell 36b on the shell skin 56, which are configured to latch into the corresponding retention hooks 72a.

Fig. 9 shows the first half-shell 36a attached to the second half-shell 36b. The retention hooks 72a of the first half-shell 36a are mounted in the counterparts 72b of the second half-shell 36b. The recess 58 is configured as a circular opening in shell skin 56.

The first fixing portion 64 of the first half-shell 36a is positioned opposite the third fixing portion 74 of the second half-shell 36b. The second fixing portion 66 of the first half-shell 36a is positioned opposite the fourth fixing portion 76 of the second half-shell 36b.

Fig. 10 shows the suction valve 26 mounted in the first fixing portion 64, the second fixing portion 66, the third fixing portion 74 and the fourth fixing portion 76. A visualization of the half-shells 36 has been omitted for the sake of clarity. The fixing projections 46 are formed on the suction valve 26, which extend radially outward away from the valve cylinder 44. The fixing projections 46 are plate-shaped geometries, which are formed with supporting ribs 82. The ribs 82 also extend radially away from the valve cylinder 44 and extend over portions of the fixing projection 46. Two ribs 82 are formed on each of the fixing projections 46.

In the assembled state, a first of the fixing projections 46 is accommodated in the second fixing portion 66. In other words, the first of the fixing projections 46 is inserted into the second fixing portion 66 in such a way that the two U-shaped brackets 68 circumferentially or peripherally surround the first of the fixing projections 46 and the first of the fixing projections 46 is in the cuboid-shaped receiving space.

The ribs 82 provided on the first of the fixing projections 46 form a stop. In other words, the first of the fixing projections 46 can only be inserted into the second fixing portion 66 until the ribs 82 abut the second fixing portion 66.

A second of the fixing projections 46 is inserted in the fourth fixing portion 76. In other words, the second of the fixing projections 46 is inserted into the fourth fixing portion 76 in such a way that the two V-shaped brackets 68 accommodate the second of the fixing projections.

Further, the suction valve 26 is mounted to the first fixing portion 64 and the third fixing portion 74. Specifically, the valve spring 50 of the suction valve 26 is connected to the housing of the handle 4 via the first fixing portion 64 and the third fixing portion 74.

The fixation of the valve spring 50 is shown in Fig. 11 and will be explained in more detail below.

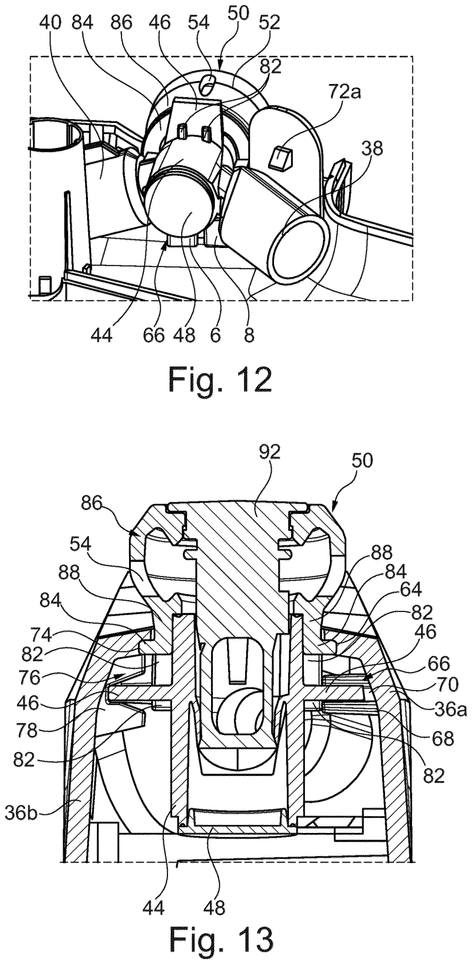

The valve spring 50 comprises a collar or flange 84 and a valve spring main body 86. The collar 84 is fixed by the first fixing portion 64 and the third fixing portion 74. The collar 84 and the valve spring main body 86 are connected to each other by a neck 88 (shown in Fig. 13). The first fixing portion 64 and the third fixing portion 74 surround the valve spring 50 in the area of the neck 88 in such a way that they fully surround the valve spring 50 in the area of the neck 88. The collar 84 is thereby positioned in the groove 62. The collar 84 comprises four tolerance absorbing bumps 90, which are each offset by 90° around the circumference of the collar 84.

In the following, the assembly of the suction valve 26 in the handle 4 is explained in more detail with reference to Figs. 12 and 13.

In a first step, shown in Fig. 12, the suction valve 26 is positioned in the first half-shell 36a. For this purpose, the first of the fixing projections 46 is inserted into the second fixing portion 66 and the neck 88 of the valve spring 50 is positioned in the first fixing portion 64. Specifically, the first of the fixing projections 46 is slid between the two U-shaped brackets 68 until the ribs 82 rest against the U-shaped brackets 68. Further, the collar 84 is positioned in the groove 62.

In a second step shown in Fig. 13, the first half-shell 36a and the second half-shell 36b are put together. Therefore, the second of the fixing projections 46 is inserted into the fourth fixing portion 76. The V-shape of the fourth fixing portion 76 makes insertion of the second of the fixing projections 46 into the fourth fixing portion 76 much easier. Furthermore, the neck 88 of the valve spring 50 is positioned in the third fixing portion 74. Therefore, the collar 84 is positioned in the groove 62. Subsequently, the handle 4 is assembled from the first half-shell 36a and the second half-shell 36b by snapping the retention hooks 72a into the counterparts 72b.

Of course, other electrical and mechanical components and assemblies may be positioned in the handle 4 prior to assembling the first half-shell 36a and the second half-shell 36b.

Fig. 14 shows a preferred embodiment of a fourth fixing portion 76. Here, the fourth fixing portion 76 also comprises two V-shaped brackets 78 (only one shown) as second tower geometries extending away from the shell skin 56 facing each other and forming a substantially V-shaped receptacle 80. The V-shaped receptacle 80 is configured to guide a fixing projection 46 of the suction valve 26 inside. A xyz-coordinate system is shown for reference.

Fig. 15 shows the fourth fixing portion 76 of Fig. 14 enlarged. The V-shaped receptacle 80 comprises a force-taking surface portion 100. A force F is shown that results from pressing or pushing the suction button 28. The force-taking surface portion 100 is an inner surface portion of the V-shaped receptacle that is perpendicular to the force F. Thereby, the force F that is (partially) transferred to the projection portion 46 is taken from the force-taking surface portion 100 providing stability. The force-taking surface portion 100 taking the brunt of the force to the V-shaped receptacle 80) prevents the suction valve 26 from moving in y-direction and rotating around the x-axis due to the force F.

Further, the V-shaped receptacle 80 comprises a flat or planar inner end surface portion 102. The end surface portion is the inner surface closest to the shell skin 56 of the second half-shell 36b. The end surface portion 102 prevents the suction valve 26 from moving (further) in z-direction.

Fig. 16 shows a preferred embodiment of a second fixing portion 66 according to the disclosure. In this embodiment, the second fixing portion 66 also comprises two U-shaped brackets 68 facing each other. The two U-shaped brackets 68 are first tower geometries protruding from the first half-shell 36a in the direction of the interior of the handle 4 and forming a split slot 104 in between them. The receptacle 70 of the second fixing portion 66 is slot-shaped. Here, the slot-shaped receptacle 70 is configured for a press-fit with the fixing projection 46 and/or they form a press-fit. A xyz-coordinate system is shown for reference.

Fig. 17 shows a sectional view of the embodiment of Fig. 16 along a cutting plane parallel to the yz-plane. One fixing projection 46 of the suction valve 26 is inserted in the receptacle 70 of the second fixing portion 66. The receptacle 70 slightly tapers in the yz-plane continuously and linearly. This enables a press-fit between the slot-shaped receptacle 70 and the fixing portion 66. The receptacle 70 has at least one angled interface also serving as an inlet chamfer.

Fig. 18 shows another sectional view of the embodiment of Fig. 16, in this case along a cutting plane parallel to the yz-plane. As shown here, the receptacle 70 also tapers continuously and linearly in this plane as a second dimension. In other words, in this preferred embodiment of Figures 16 to 18, the receptacle has a rectangular cross-section tapering, i.e. becoming narrower in width and height further inside the receptacle.

Comparing the preferred embodiments of the V-shaped receptacle 80 of Fig. 14 and the slot-shaped receptacle 70 of Fig. 16, it is apparent that in this case the second, inner fixing portion 66 with the slot-shaped receptacle 70 suppresses more degrees of freedom of the suction valve 26 than the fourth, inner fixing portion 76 with V-shaped receptacle 80.

Due to the shape of the slot-shaped receptacle 70 the second, inner fixing portion 66 is configured or shaped to suppress translational movements of the suction valve 26 relative to the housing of the endoscope except in direction of the second half-shell 36b for disassembly when the fixing projection 46 is inserted in an assembled state of the endoscope, wherein the fourth, inner fixing portion 76 with the V-shaped receptacle 80 is configured/ shaped to prevent this movement and due its shape is configured to guide the suction valve in that direction inwards during assembly, making the assembly easier.

The slot-shaped receptacle 70 of the second, inner fixing portion 66 is configured to suppress by its shape translational movements and (arbitrary) rotational movements of the suction valve 26 relative to the housing of the endoscope, except for translational movements in direction of the second half-shell 36b, so that it is possible to take the fixing projection 46 out of the receptacle during disassembly of the endoscope 2. It provides with its shape due to the angled interfaces some small rotational looseness or clearance for the fixing projection 46 making the assembly easier. that the rotational looseness or clearance may be suppressed by the press-fit.

### List of reference numbers

- 2: endoscope
- 4: endoscope handle
- 6: insertion cord
- 8: insertion tube
- 10: bending section
- 12: distal tip unit
- 14: working channel
- 16: working channel access port
- 18: working channel tube
- 20: operating unit
- 22: Y-connector
- 24: connecting cable
- 24a: electrical connector
- 26: suction valve
- 28: suction button
- 30: suction channel
- 32: vacuum port
- 34: hose
- 36: half-shell
- 36a: first half-shell
- 36b: second half-shell
- 38: inlet
- 40: outlet
- 42: valve body
- 44: valve cylinder
- 46: fixing projection
- 48: end cap
- 50: valve spring
- 52: side surface
- 54: bypass hole
- 56: shell skin
- 58: recess
- 60: contact edge
- 62: groove
- 64: first fixing portion
- 66: second fixing portion
- 68: U-shaped bracket
- 70: slot-shaped receptacle
- 72a: retention hook
- 72b: counterpart
- 74: third fixing portion
- 76: fourth fixing portion
- 78: V-shaped bracket
- 80: V-shaped receptacle
- 82: rib
- 84: collar/ flange
- 86: spring main body
- 88: neck
- 90: bump
- 92: stem
- A1: first center axis
- A2: second center axis
- α: angle
- P: suction pump
- VPA: video processing apparatus
- 100: force-taking surface portion
- 102: end surface portion
- 104: split slot

## Claims

1. An endoscope (2) comprising:
a proximal endoscope handle or interface (4);
an insertion cord (6) extending from the endoscope handle or interface (4) and configured to be inserted into a patient's body cavity;
a working channel (14) configured to let fluid and mucus sucked from the patient's body cavity flow therethrough;
the endoscope handle or interface (4) comprising:
a housing; and
a suction valve (26) configured to control a suction through the working channel (14) and having a valve closed state and a valve open state;
the housing comprising a first half-shell (36a) and a second half-shell (36b);
the suction valve (26) comprising:
a valve body (42) having:
an inlet connected (38) to the working channel (14);
an outlet (40) provided and configured to be connected to a suction device (P);
and
a stem (92) movable axially in the valve body (42) between at least a first position, in which the suction valve (26) is in the valve closed state, and a second position, in which the suction valve is in the valve open state;
wherein the suction valve (26) is mounted between the first half-shell (36a) and the second half-shell (36b); and wherein
the first half-shell (36a) comprises
a first, outer fixing portion (64) and
a second, inner fixing (66) portion,
wherein the first, outer fixing portion (64) is configured to receive a first portion of the suction valve (26) and wherein the second, inner fixing portion (66) is configured to receive a second portion of the suction valve (26),
the second half-shell (36b) comprises
a third, outer fixing portion (74) and
a fourth, inner fixing portion (76),
wherein the third, outer fixing portion (74) is configured to receive the first portion of the suction valve (26), and wherein the fourth, inner fixing portion (76) is configured to receive the second portion of the suction valve (26),
wherein the second, inner fixing portion (66) of the first half shell (36a) and the fourth inner fixing portion (76) of the second half shell (36b) have a different geometry, and
wherein the second, inner fixing portion (66) is configured to suppress at least one degree of freedom of the suction valve (26) and the fourth, inner fixing portion (76) is configured to suppress a remaining degree or remaining degrees of freedom of the suction valve (26), for fixing the suction valve in its position and orientation in the housing.

2. The endoscope (2) according to claim 1, wherein the second, inner fixing portion (66) is configured to suppress more degrees of freedom of the suction valve (26) than the fourth, inner fixing portion (76).

3. The endoscope (2) according to claim 1 or 2, wherein the second, inner fixing portion (66) is configured to suppress by its shape translational movements and arbitrary rotational movements of the suction valve (26) relative to the housing of the endoscope handle or interface (4) except for a translational movement in direction of the second half-shell (36b), and wherein the fourth, inner fixing portion (76) is configured to suppress the translational movement in direction of the second half-shell (36b).

4. The endoscope (2) according to any of claims 1 to 3, wherein the second, inner fixing portion (66) of the first half-shell (36a) comprises a slot-shaped receptacle (70) configured to hold a fixing projection (46) of the suction valve (26).

5. The endoscope (2) according to claim 4, wherein the slot-shaped receptacle (70) of the second, inner fixing portion (66) tapers and forms a press-fit with the fixing projection (46) of the suction valve (26).

6. The endoscope (2) according to claim 4 or 5, wherein the second, inner fixing portion (66) comprises two U-shaped brackets (68) that form the slot-shaped receptacle (70) and a split slot (104) in between them.

7. The endoscope (2) according to any of claims 1 to 6, wherein the fourth, inner fixing portion (76) of the second half-shell (36b) comprises a V-shaped receptacle (80) configured to guide in a fixing projection (46) of the suction valve (26) during assembly.

8. The endoscope (2) according to claim 7, wherein the V-shaped receptacle (80) comprises a flat force-taking surface portion (100) perpendicular to a direction in which a button (28) of the suction valve (26) is pushable to move the stem (92), so that when the button (28) is pushed, a movement of the suction valve (26) in that direction relative to the housing is suppressed by the flat force-taking surface portion (100).

9. The endoscope (2) according to any of claims 1 to 8, wherein the second, inner fixing portion (66) of the first half-shell (36a) comprises a slot (70), preferably of constant width, and the fourth, inner fixing portion (76) of the second half-shell (36b) comprises comprises a V-shaped slot (80).

10. A method of assembling an endoscope (2) according to any of claims 1 to 9, comprising the steps:
Sliding the valve spring (50) onto the valve body (42) of the suction valve (26) and latching the valve spring (50) to the stem (92) of the suction valve (26);
Positioning the suction valve (26) in the first half-shell (36a) of the housing of an endoscope handle or interface (4) by inserting the second portion of the suction valve (26) in the second, inner fixing portion (66) of the first half-shell (36a) of the housing;
Inserting the first portion of the suction valve (26) in the first, outer fixing portion (64) of the first half-shell (36a);
Positioning the second half-shell (36b) of the housing on the first half-shell (36a) of the housing;
Inserting the second portion of the suction valve (26) in the fourth, inner fixing portion (76) of the second half-shell (36b) of the housing;
Inserting the first portion of the suction valve (26) in the third, outer fixing portion (74) of the second half-shell (36b);
Closing the half-shells (36a; 36b).

11. A system comprising an endoscope (2) according to one of claims 1 to 9 and a video processing apparatus (VPA).
